(19) Europäisches Patentamt
European Patent Office
Office européen des brevets

(11) Veröffentlichungsnummer: **0 155 931**
**A2**

(12) # EUROPÄISCHE PATENTANMELDUNG

(21) Anmeldenummer: 85890065.7

(22) Anmeldetag: 19.03.85

(51) Int. Cl.⁴: **C 07 H 15/04**
C 08 B 31/12

(30) Priorität: 20.03.84 AT 934/84

(43) Veröffentlichungstag der Anmeldung:
25.09.85 Patentblatt 85/39

(84) Benannte Vertragsstaaten:
BE CH DE FR GB IT LI LU NL SE

(71) Anmelder: Evidenzbüro österreichischer Zuckerfabriken Gesellschaft m.b.H.
Zaunergasse 1-3
A-1031 Wien(AT)

(72) Erfinder: Greber, Gerd, Dr.
Anzengruberstrasse 11
A-2540 Bad Vöslau(AT)

(72) Erfinder: Gruber, Heinrich, Dr.
Cottagegasse 82
A-1190 Wien(AT)

(74) Vertreter: Pfeifer, Otto, Dipl.-Ing. et al,
Patentanwälte Dipl.-Ing. Dr. techn. Schütz, Alfred Dr. phil. Mrazek, Engelbert Dipl.-Ing. Holzer, Walter Dipl.-Ing. Pfeifer, Otto Fleischmanngasse 9
A-1040 Wien(AT)

(54) **Kohlenhydrat-Derivate mit beta-Nitroethyl- bzw. beta-Aminoethyl Gruppen und Verfahren zu deren Herstellung.**

(57) Es werden neue Kohlenhydrat-Derivate mit etherartig gebundenen β-Nitroethyl- bzw. β-Aminoethyl-Gruppen der allgemeinen Formel (I)

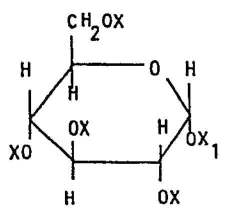

(I)

beschrieben, worin $X_1$ für H, X, $CH_3$, $C_2H_5$, $CH_2CH_2OH$, $CH_2CH(OH)CH_3$, $CH_2CH(OH)CH_2OH$

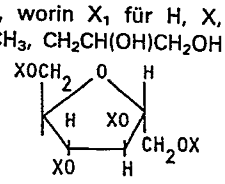

oder Reste löslicher Stärken mit mittleren Molekulargewichten von 1000 bis etwa 30000 steht und mindestens ein X für etherartig gebundene β-Nitroethyl- bzw. β-Aminoethyl-Gruppen der allgemeinen Formeln (II) und (III)

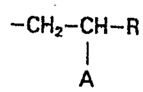

(II)

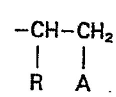

(III)

steht, worin A eine Nitrogruppe oder eine Aminogruppe bedeutet und R $-CH_3$, $-(CH_2)_n-CH_3 (n=1-8)$ oder einen verzweigten aliphatischen Rest mit 3 bis 10 C-Atomen bedeutet und die restlichen X für Wasserstoff oder Acylreste mit 1 bis 20 C-Atomen, insbesondere Acetylreste, stehen. Die Herstellung der neuen Kohlenhydrat-Derivate (I) kann durch Umsetzung entsprechender Kohlenhydrate mit 1- oder 2-Nitroolefinen und gegebenenfalls anschließende Reduktion zu den Aminoverbindungen erfolgen.

Die neuen Kohlenhydrat-Derivate (I) sind interessante Zwischenprodukte für weitere Synthesen, etwa zur Gewinnung hydrolysebeständiger Amid-Derivate.

EP 0 155 931 A2

0155931

# Kohlenhydrat-Derivate mit ß-Nitroethyl- bzw. ß-Aminoethyl-Gruppen und Verfahren zu deren Herstellung

Die Erfindung betrifft neue Kohlenhydrat-Derivate mit ß-Nitroethyl- bzw. ß-Aminoethyl-Gruppen sowie Verfahren zu deren Herstellung.

Das chemische Verhalten von Kohlenhydraten wie z.B. Mono- und Disacchariden, Glukosiden, Stärken oder kondensierten Polyglukosen, wird vor allem durch die Anhäufung von alkoholischen Hydroxylgruppen geprägt: sie bedingen einen ausgeprägt hydrophilen Charakter und ermöglichen eine Reihe von Reaktionen. Allerdings werden die Umsetzungsmöglichkeiten sowohl durch die beschränkte Löslichkeit in organischen Lösungsmitteln als auch durch eine relativ geringe chemische Beständigkeit in gewissem Umfang begrenzt. Das größte technische Interesse haben bisher vor allem Ester- und Ether-Derivate gefunden, wobei die Substituenten je nach Einsatzgebiet - etwa Waschmittel, Kosmetika, Klebstoffe usw . - meist aus inerten langkettigen aliphatischen, araliphatischen, aromatischen oder anderen hydrophobierenden organischen Resten bestehen. Für bestimmte Anwendungsgebiete - beispielsweise für oberflächenaktive Substanzen - benötigt man oft einheitliche Substitutionsprodukte. Da jedoch die Reaktivität der Hydroxylgruppen von unsubstituieren im Vergleich mit teilweise substituierten Kohlenhydraten geringer ist, entstehen bei solchen Reaktionen immer Gemische verschieden substituierter - gegebenenfalls noch isomerer - Derivate, aus denen einheitliche Produkte nur mit großem Aufwand und in geringen Ausbeuten isolierbar sind. Diese Problematik hat bisher den großtechnischen Einsatz von Saccharose-Derivaten weitgehend blockiert.

Im Vergleich zu solchen Ester- und Ether-Derivaten ist dagegen wenig bekannt über Kohlenhydrat-Derivate, bei denen die Substituenten - die ebenfalls etherartig gebunden sein können - eine zusätzliche hochreaktive Gruppe enthalten, die weitere Umsetzungen ermöglicht.

Gegenstand der Erfindung sind neue Kohlenhydrat-Derivate mit etherartig gebundenen ß-Nitroethyl- bzw. ß-Aminoethyl-Gruppen der allgemeinen Formel (I)

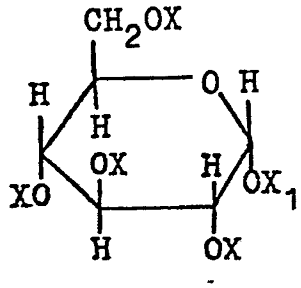, (I)

worin $X_1$ = H, X, $CH_3$, $C_2H_5$, $CH_2CH_2OH$, $CH_2CH(OH)CH_3$,

$CH_2CH(OH)CH_2OH$, 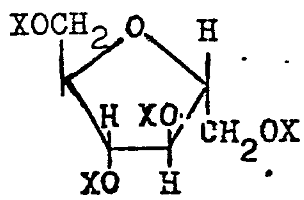

oder Reste löslicher Stärken mit mittleren Molekulargewichten von 1000 bis etwa 30000 bedeutet und mindestens ein X für etherartig gebundene ß-Nitroethyl- bzw. ß-Aminoethyl-Gruppen der allgemeinen Formeln (II) und (III)

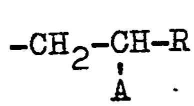 (II) oder 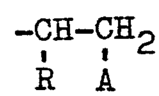 (III)

steht, worin A eine Nitrogruppe oder eine Aminogruppe bedeutet und R $-CH_3$, $-(CH_2)_n-CH_3$(n=1-8) oder verzweigte aliphatische Reste mit 3 bis 10 C-Atomen bedeutet und die restlichen X für Wasserstoff oder Acylreste mit 1 bis 20 C-Atomen, insbesondere Acetylreste, stehen.

Die neuen Kohlenhydrat-Derivate der Formel (I) mit ß-Nitroethyl-Gruppen erhält man erfindungsgemäß durch Umsetzung der der Formel (I) zugrundeliegenden unsubstituierten Kohlenhydrat-Derivate mit 1- oder 2-Nitroolefinen der Formeln (IIa) und (IIIa) in wässerigem Milieu in Gegenwart von basischen Katalysatoren:

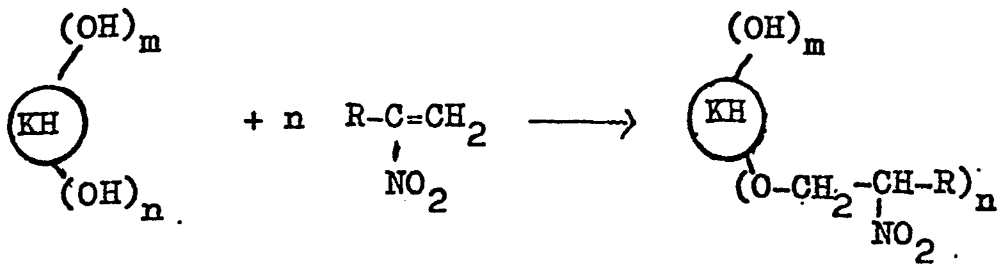

(IIa)

bzw.

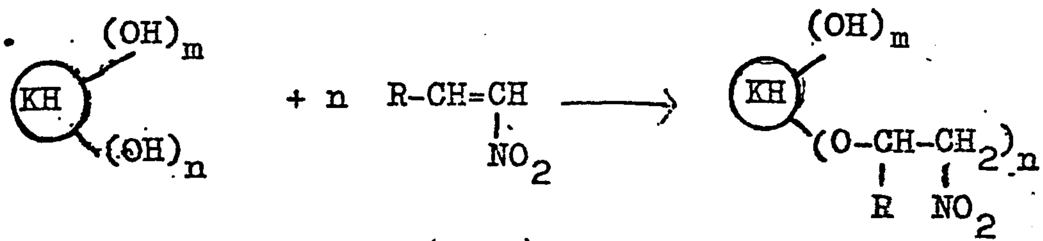

KH = Kohlenhydrat (IIIa)

Die Umwandlung der ß-Nitroethyl-Gruppen in ß-Aminoethyl-Gruppen erfolgt durch katalytische Hydrierung mit den üblichen Katalysatoren in an sich bekannter Weise.

Es ist bekannt, daß man Alkohol unter basischer Katalyse an aktivierte 1- bzw. 2-Nitroolefine addieren kann, wobei die entsprechenden isomeren ß-Nitro-alkoxy-Derivate entstehen. Diese nukleophile Additionsreaktion ist als Michael-Reaktion bekannt. In der Literatur sind solche Reaktionen bisher vor allem mit niederen einwertigen Alkoholen ($C_1$ bis $C_5$) beschrieben, nur vereinzelt wird auch über die Addition von Nitroolefinen an Ethylenglykol und Propylenglykol berichtet (vgl. Houben-Weyl, Bd.10/1 S. 402 ff. und dort zitierte Literatur). Über die Addition von mehrwertigen Alkoholen und Kohlenhydraten an Nitroolefine ist dagegen bisher nichts bekanntgeworden.

Die Überführung der Nitroolefine in die Ether erfordert in der Regel die Anwesenheit von überschüssigem Alkalimetallalkoholat und den entsprechenden wasserfreien Alkohol als Lösungsmittel. Diese Bedingungen können bekanntlich bei Kohlenhydraten nicht angewandt werden, da es sich einmal durchwegs um Feststoffe handelt und zum andern die entsprechenden Alkoholate nicht hergestellt werden können.

Überraschenderweise wurde nun gefunden, daß die Addition von Kohlenhydraten an Nitroolefine auch in Wasser als Lösungsmittel und in Gegenwart basischer Katalysatoren durchgeführt werden kann, wobei je nach Molverhältnis der Reaktionspartner Gemische von mono- und polyfunktionellen ß-Nitroethylethern entstehen. Die neuen Kohlenhydrat-Derivate mit etherartig gebundenen ß-Nitroethyl-Gruppen werden erfindungsgemäß dadurch erhalten, daß man die entsprechenden Kohlenhydrate mit einer dem gewünschten mittleren Substitutionsgrad entsprechenden Menge an Nitroolefin in Gegenwart der stöchiometrischen Menge einer basischen Verbindung, beispielsweise einem Alkalihydroxid, in wässeriger Lösung umsetzt.

Zweckmäßig wird das Kohlenhydrat-Derivat in 10-20 %iger wässeriger Lösung des basischen Katalysators gelöst. Die Temperatur wird auf zirka 20°C eingestellt und die Geschwindigkeit der Zugabe des Nitroolefins vorteilhaft so eingerichtet, daß die Reaktionstemperatur 30°C nicht überschreitet. Das Reaktionsgemisch wird anschließend mit verdünnter Säure auf pH 5 eingestellt, das Wasser abdestilliert und der Rückstand in einem organischen Lösungsmittel - vorzugsweise Ethanol, n- oder iso-Propanol oder Aceton - aufgenommen. Nach Abfiltrieren oder Abzentrifugieren der unlöslichen Salze erhält man die Endprodukte durch Abdestillieren des Lösungsmittels und Vakuumtrocknung des Rückstandes. Eine andere Möglichkeit der Aufarbeitung besteht darin, die wässerige Lösung mit einem geeigneten Lösungsmittel zu extrahieren. Hierzu eignen sich - in Abhängigkeit vom mittleren Substitutionsgrad - z.B. halogenierte Kohlenwasserstoffe oder Diethylether.

Als basische Katalysatoren können beispielsweise Alkalihydroxide, Alkalibicarbonate, Tetraalkylammoniumhydroxide oder stark basische Ionenaustauscher eingesetzt werden.

Je nach der eingesetzten Menge an Nitroolefin erhält man, beispielsweise bei der Saccharose, mittlere Substitutionsgrade von 1 bis 6. ß-Nitroethyl-Saccharose-Derivate mit Substitutionsgraden bis etwa 3 sind gelbliche Farbstoffe, die in Wasser und polaren organischen Lösungsmitteln löslich sind. Derivate mit höheren Substitutionsgraden sind meist zähflüssige Substanzen, die wasserunlöslich und in polaren Lösungsmitteln löslich sind. Bei anderen Kohlenhydraten - beispielsweise Glukose, Alkyl- und Arylglukosiden und löslichen Stärken - findet man mittlere Substitutionsgrade bis etwa 80 % der vorhandenen Hydroxylgruppen.

Geeignete Nitroolefine sind beispielsweise 2-Nitropropen, 2-Nitrobuten-1, 1-Nitrobuten-1, 1-Nitropenten-1 und 1-Nitroocten-1.

Nitroolefine können nach literaturbekannten Verfahren hergestellt werden, beispielsweise durch Eliminierung von Essigsäure aus α-Nitro-ß-acetoxy-alkanen oder Abspaltung von Halogenwasserstoff aus α-Nitro-ß-halogenalkanen (vgl.Houben-Weyl Bd.10/1, S. 330 ff):

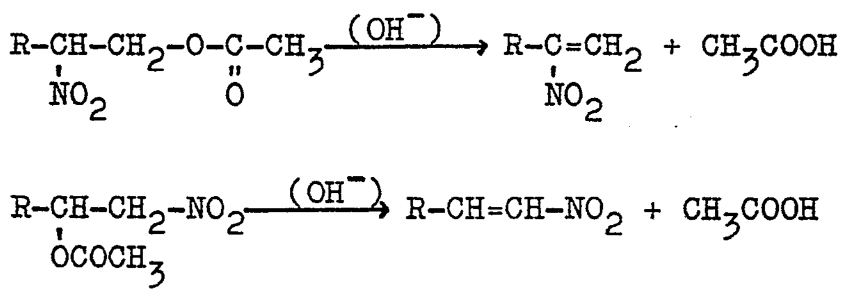


Die Reaktion kann vorteilhaft auch so durchgeführt werden, daß man anstelle der Nitroolefine Verbindungen einsetzt, aus denen im Reaktionsgemisch Nitroolefine in situ gebildet werden, beispielsweise 2-Nitroalkylacetate. In diesem Fall ist ein zusätzlicher Katalysator, beispielsweise Natriumacetat erforderlich; ansonsten wird die Reaktion wie beschrieben durchgeführt und aufgearbeitet.

Die Kohlenhydrat-Derivate der Formel (I) mit ß-Nitroethyl-Gruppen können in an sich bekannter Weise zu den entsprechenden ß-Aminoethyl-Derivaten hydriert werden, beispielsweise in

Methanol, Ethanol, Isopropanol oder anderen geeigneten organischen Lösungsmitteln mit Katalysatoren wie Raney-Ni, Pt-Aktivkohle oder Platinoxid bei 50-100°C und einem Wasserstoffdruck von 10-100 bar.

ß-Aminoethyl-Kohlenhydratderivate sind je nach Substitutionsgrad feste oder zähflüssige Substanzen. Die primären Aminogruppen können in an sich bekannter Weise durch Titration mit Perchlorsäure in Eisessig bestimmt werden.

Die erfindungsgemäß hergestellten Kohlenhydrat-Derivate mit ß-Nitroethyl- bzw. ß-Aminoethyl-Gruppen sind interessante Zwischenprodukte für weitere Synthesen, etwa zur Gewinnung hydrolysebeständiger Amid-Derivate.

In den folgenden Beispielen wird die Herstellung der neuen Kohlenhydrat-Derivate näher erläutert.

Beispiel 1:

3,42 g (0,01 Mol) Saccharose und 1,68 g (0,03 Mol) KOH werden in 15 ml Wasser gelöst. Dann werden unter Rühren 3,06 g (0,03 Mol) 2-Nitrobuten-1 so zugetropft, daß die Temperatur nicht über 30°C steigt. Anschließend wird noch 6 Stunden bei Raumtemperatur gerührt. Das Reaktionsgemisch wird mit verdünnter Salzsäure auf pH 5 eingestellt und am Rotationsverdampfer zur Trockene eingedampft. Der Rückstand wird in 50 ml Ethanol gerührt, abfiltriert und die alkoholische Lösung wird zur Trockene eingedampft. Der Rückstand wird bei 40°C im Vakuum getrocknet.

Ausbeute: 5,7 g ; durchschnittlicher Substitutionsgrad (DS) 1,8 (berechnet aus dem N-Gehalt)

Beispiel 2:

Die Vorgangsweise von Beispiel 1 wird wiederholt, mit dem Unterschied, daß 3,42 g (0,01 Mol) Saccharose, 1,12 g (0,02 Mol) KOH und 2,04 (0,02 Mol) 2-Nitrobuten-1 eingesetzt werden.

Ausbeute: 4,6 ; DS 1,2

Beispiel 3:

Die Vorgangsweise von Beispiel 1 wird wiederholt, mit dem Unterschied, daß 3,42 g (0,01 Mol) Saccharose, 2,8 g (0,05 Mol)

KOH und 5,1 g (0,05 Mol) 2-Nitrobuten-1 eingesetzt werden.

Ausbeute: 6,5 g ; DS 3,9

Beispiel 4:

Die Vorgangsweise von Beispiel 1 wird wiederholt, mit dem Unterschied, daß 3,42 g (0,01 Mol) Saccharose, 3,92 g (0,07 Mol) KOH und 7,14 g (0,07 Mol) 2-Nitrobuten-1 eingesetzt werden.

Ausbeute: 9,8 ; DS 4,6

Beispiel 5:

Die Vorgangsweise von Beispiel 1 wird wiederholt, mit dem Unterschied, daß 20,56 g (0,06 Mol) Saccharose, 10,1 g (0,18 Mol) KOH und 18,36 g (0,18 Mol) 2-Nitrobuten-1 und 80 ml Wasser eingesetzt werden. In der Aufarbeitung werden 200 ml Ethanol eingesetzt.

Ausbeute: 35 g ; DS 1,7

Beispiel 6:

Die Vorgangsweise von Beispiel 1 wird wiederholt, mit dem Unterschied, daß 3,42 g (0,01 Mol) Saccharose, 1,68 g (0,03 Mol) KOH und 2,64 g (0,03 Mol) 2-Nitropropen eingesetzt werden.

Ausbeute: 5,2 g ; DS 2,0

Beispiel 7:

Die Vorgangsweise von Beispiel 1 wird wiederholt, mit dem Unterschied, daß 3,42 g (0,01 Mol) Saccharose, 1,68 g (0,03 Mol) KOH und 3,45 g (0,03 Mol) 1-Nitropenten-1 eingesetzt werden.

Ausbeute: 4,8 g ; DS 1,6

Beispiel 8:

Die Vorgangsweise von Beispiel 1 wird wiederholt, mit dem Unterschied, daß 3,42 g (0,01 Mol) Saccharose, 1,68 g (0,03 Mol) KOH und 4,71 g (0,03 Mol) 1-Nitroocten-1 eingesetzt werden.

Ausbeute: 5,3 g ; DS 1,5

Beispiel 9:

3,42 g (0,01 Mol) Saccharose, 1,68 g (0,03 Mol) KOH und 4,1 g (0,03 Mol) Natriumacetat.3 $H_2O$ werden in 15 ml Wasser gelöst und 4,83 g (0,03 Mol) 2-Nitrobutylacetat unter Rühren so zugetropft, daß die Temperatur nicht über 30°C steigt. Anschließend wird noch 6 Stunden bei Raumtemperatur gerührt. Das Reaktionsgemisch wird mit verdünnter Salzsäure auf pH 5 eingestellt und am Rotationsverdampfer zur Trockene eingedampft. Der Rückstand wird in 50 ml Ethanol gerührt, abfiltriert und die alkoholische Lösung wird zur Trockene eingedampft. Der Rückstand wird bei 40°C im Vakuum getrocknet.

Ausbeute: 5,9 g; DS 1,8

Beispiel 10:

Die Vorgangsweise von Beispiel 9 wird wiederholt, mit dem Unterschied, daß 3,42 g (0,01 Mol) Saccharose, 2,8 g (0,05 Mol) KOH, 6,85 g (0,05 Mol) Natriumacetat.3 $H_2O$ und 8,05 g (0,05 Mol) 2-Nitrobutylacetat eingesetzt werden.

Ausbeute: 6,4 g; DS 3,8

Beispiel 11:

Die Vorgangsweise von Beispiel 9 wird wiederholt, mit dem Unterschied, daß 3,42 g (0,01 Mol) Saccharose, 1,68 g (0,03 Mol) KOH, 4,2 g (0,03 Mol) Natriumacetat.3 $H_2O$ und 4,41 g (0,03 Mol) 2-Nitropropylacetat eingesetzt werden.

Ausbeute: 5,4 g; DS 1,9

Beispiel 12:

1,71 g (0,005 Mol) Saccharose werden in 10 ml Wasser gelöst, 3 g stark basischer Ionenaustauscher (Merck, Typ III, Kapazität zirka 5 mval/g) zugegeben und unter Rühren 1,31 g (0,015 Mol) 2-Nitropropen zugetropft, so daß die Temperatur nicht über 30°C steigt. Anschlißend wird noch 8 Stunden bei Raumtemperatur gerührt. Der pH-Wert der Lösung wird auf 5 eingestellt und der Ionenaustauscher wird abfiltriert. Die Lösung wird am Rotationsverdampfer zur Trockene eingedampft und der Rückstand wird im Vakuum getrocknet.

Ausbeute: 2,5 g; DS 1,7

Beispiel 13:

1,8 g Glucose (0,01 Mol) und 1,68 g (0,03 Mol) KOH werden in 10 ml Wasser gelöst. Dann werden unter Rühren 2,61 g (0,03 Mol) 2-Nitropropen so zugetropft, daß die Temperatur nicht über 30°C steigt. Anschließend wird noch 6 Stunden bei Raumtemperatur gerührt. Das Reaktionsgemisch wird mit verdünnter Salzsäure auf pH 5 eingestellt undam Rotationsverdampfer zur Trockene eingedampft. Der Rückstand wird in 30 ml Ethanol gerührt, abfiltriert und die alkoholische Lösung wird zur Trockene eingedampft. Der Rückstand wird bei 40°C im Vakuum getrocknet.

Ausbeute: 3,6 g; DS 1,9

Beispiel 14:

Die Vorgangsweise von Beispiel 13 wird wiederholt, mit dem Unterschied, daß 1,94 g (0,01 Mol) Methylglucosid, 1,68 g (0,03 Mol) KOH und 2,61 g (0,03 Mol) 2-Nitropropen eingesetzt werden.

Ausbeute: 3,8 g; DS 2,2

Beispiel 15:

Die Vorgangsweise von Beispiel 13 wird wiederholt, mit dem Unterschied, daß 1,62 g (0,01 Mol) Polydextrose (= synthet. hergestellt durch Kondensation von Glucose mit Sorbit und Zitronensäure, MG zirka 1500, Fa. Pfizer), 1,12 g (0,02 Mol) KOH und 1,74 g (0,02 Mol) 2-Nitropropen eingesetzt werden.

Ausbeute: 2,6 g; DS 1,3

Beispiel 16:

1,62 g (0,01 Mol) lösliche Stärke und 1,12 g (0,02 Mol) KOH werden in 10 ml Wasser gelöst. Dann werden unter Rühren 1,74 g (0,02 Mol) 2-Nitropropen so zugetropft, daß die Temperatur nicht über 30°C steigt. Anschließend wird noch 6 Stunden bei Zimmertemperatur gerührt. Das Reaktionsgemisch wird mit verdünnter Salzsäure auf pH 5 eingestellt und das Produkt wird

mit zirka 20 ml Ethanol ausgefällt. Nach dem Abfiltrieren wird im Vakuum bei 50°C getrocknet.

Ausbeute: 1,9 g; DS 1,1

Beispiel 17:

2 g Saccharose-2-nitrobutylether-Gemisch mit DS 1,8 (vgl. Beispiel 1) werden in 50 ml Ethanol gelöst, 0,3 g Platin-Aktivkohle-Katalysator zugegeben und das Gemisch wird bei 40 bar Wasserstoff und 60°C 16 Stunden lang im Autoklaven gerührt. Dann wird der Katalysator abfiltriert und das Ethanol abdestilliert. Der Rückstand wird im Vakuum bei 40°C getrocknet.

Ausbeute: 1,9 g; DS 1,6 (berechnet aus der Elementaranalyse und aus der Titration der Aminogruppen)

Beispiel 18:

Die Vorgangsweise von Beispiel 17 wird wiederholt, mit dem Unterschied, daß 2 g Saccharose-2-nitrobutylether mit DS 3,9 (vgl. Beispiel 3) eingesetzt werden.

Ausbeute: 1,8 g; DS 3,1

Beispiel 19:

Die Vorgangsweise von Beispiel 17 wird wiederholt, mit den Unterschied, daß 2 g Saccharose-2-nitrobutylether-Gemisch mit DS 1,8 und 0,1 g Platinoxid-Katalysator eingesetzt werden.

Ausbeute: 1,9 g; DS 1,6

Beispiel 20:

Die Vorgangsweise von Beispiel 17 wird wiederholt, mit dem Unterschied, daß 0,5 g Raney-Nickel als Katalysator eingesetzt werden.

Ausbeute: 1,9 g; DS 1,6.

Patentansprüche:

1. Kohlenhydrat-Derivate mit etherartig gebundenen ß-Nitroethyl- bzw. ß-Aminoethyl-Gruppen der allgemeinen Formel (I)

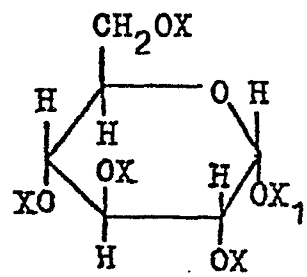

, (I)

worin $X_1$ für H, X, $CH_3$, $C_2H_5$, $CH_2CH_2OH$, $CH_2CH(OH)CH_3$, $CH_2CH(OH)CH_2OH$,

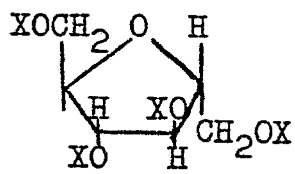

oder Reste löslicher Stärken mit mittleren Molekulargewichten von 1000 bis etwa 30000 steht und mindestens ein X für etherartig gebundene ß-Nitroethyl- bzw. ß-Aminoethyl-Gruppen der allgemeinen Formeln (II) und (III)

$-CH_2-\underset{A}{CH}-R$ (II) $-\underset{R}{CH}-\underset{A}{CH_2}$ (III)

steht, worin A eine Nitrogruppe oder eine Aminogruppe bedeutet und R $-CH_3$, $-(CH_2)_n-CH_3 (n=1-8)$ oder einen verzweigten aliphatischen Rest mit 3 bis 10 C-Atomen bedeutet und die restlichen X für Wasserstoff oder Acylreste mit 1 bis 20 C-Atomen, insbesondere Acetylreste, stehen.

2. Verfahren zur Herstellung von neuen Kohlenhydrat-Derivaten mit ß-Nitroethylgruppen der Formel (I), dadurch gekennzeichnet, daß man die den Kohlenhydrat-Derivaten der Formel (I) zugrundeliegenden Kohlenhydrate mit 1- oder 2-Nitroolefinen der Formeln

$CH_2=C-R$ bzw. $HC=CH$

$NO_2$ $R$ $NO_2$

(IIa) (IIIa)

worin R die in Anspruch 1 angegebene Bedeutung hat, in wässeriger Lösung und in Gegenwart alkalischer Katalysatoren umsetzt.

3. Verfahren zur Herstellung von neuen Kohlenhydrat-Derivaten mit ß-Aminoethyl-Gruppen der Formel (I), dadurch gekennzeichnet, daß man erhaltene Kohlenhydrat-Derivate der Formel (I) mit ß-Nitroethylgruppen in Gegenwart bekannter Hydrierungskatalysatoren hydriert.

4. Verfahren nach Anspruch , dadurch gekennzeichnet, daß man von Saccharose als Kohlenhydrat ausgeht.

5. Verfahren nach Anspruch 2 oder 4, dadurch gekennzeichnet, daß man als alkalischen Katalysator ein Alkalihydroxid oder einen stark basischen Ionenaustauscher einsetzt.

6. Verfahren nach einem der Ansprüche 2, 4 und 5, dadurch gekennzeichnet, daß man das Nitroolefin im Reaktionsgemisch in situ ausbildet.

7. Verfahren nach Anspruch 6, dadurch gekennzeichnet, daß man als Nitroolefin-Vorstufe das entsprechende 2-Nitroalkylacetat und Natriumacetat einsetzt.

8. Verfahren nach Anspruch 3, dadurch gekennzeichnet, daß man die Hydrierung über Raney-Nickel, Platin/Aktivkohle oder Platinoxid bei 50-100°C und einem Wasserstoffdruck von 10 bis 100 bar vornimmt.

DI.Pf/Fr 1985 03 01/